# EUROPEAN PATENT APPLICATION

(11) **EP 3 705 095 A1**
(43) Date of publication of application: **09.09.2020**
(21) Application number: 17929325.3
(22) Date of filing: 08.11.2017
(51) Int. Cl.: A61F 9/00

(54) **FLUID SUPPLY UNIT, AND MICRO-DROPLET EJECTION DRIVING DEVICE AND GENERATING DEVICE**

(30) Priority: 19.10.2017 CN 201710980387
(71) Applicant: Shenzhen Qiming Pharmaceutical Technology Co., Ltd, Shenzhen, Guangdong 518000 (CN)
(72) Inventor: LI, Huanchang, Shenzhen Guangdong 518000 (CN); FAN, Biao, Shenzhen Guangdong 518000 (CN)
(74) Representative: Cueto, Sénida
(86) International application number: PCT/CN2017/109817
(87) International publication number: WO 2019/075802

(57) **Abstract**

Provided is a fluid supply unit (2), a micro-droplet ejection driving device (1) and a micro-droplet ejection generating device (4). The fluid supply unit (2) comprises a fluid ejecting portion (210) and an energy conducting sheet (220), the fluid ejecting portion (210) and the energy conducting sheet (220) constituting at least part of a container wall of a container to be injected with a fluid; the energy conducting sheet (220) is used in close contact with an end surface of a piezoelectric actuator (120) and is driven to generate vibrations, thereby causing the fluid to be ejected by means of the fluid ejecting portion so as to form a directional micro-droplet stream. The micro-droplet ejection driving device (1) comprises: a housing (110) in which the fluid supply unit (2) may be accommodated; the piezoelectric actuator (120), which is fixed on the housing (110) and which is configured to be in close contact with an outer wall of the fluid supply unit (2) and to drive the outer wall to vibrate. The micro-droplet ejection generating device (4) comprises the fluid supply unit (2) and the micro-droplet ejection driving device (1).

## Description

### TECHNICAL FIELD

The present invention relates to the technical field of droplet ejection, and more particularly, to a fluid supply unit for ejecting, a micro-droplet ejection driving device, and a generating device.

### BACKGROUND

A commonly used droplet applicator uses a method of dropping a drug droplet into a corresponding part of the body for administration. It has many disadvantages, such as the medicinal solution cannot reach the affected area effectively, or the dose of the medicine cannot be accurately controlled. Taking the use of drop-on eye drops as an example, since the squeezed drop of drug drops is relatively large, the user may blink involuntarily, resulting in that many drops cannot reach the eyes effectively. Therefore, the traditional way of administering liquid droplets cannot control the amount of medicine used on the one hand, which causing waste; on the other hand, it is not comfortable for the users.

In order to increase the efficiency and convenience of drug administration, a microfluid ejecting drug delivery device has been developed. It connects the medicine supply device with the atomization device through the connection device. Once used, there will be drug residues in the ejection chamber of the atomization device, so it cannot be used for a long time. Since the ejection driving element of the atomizing device and the main body have an integral connection structure, they can only be discarded integrally after used.

### SUMMARY

A brief overview of the disclosure is given below in order to provide a basic understanding of certain aspects of the disclosure. It should be understood that this summary is not an exhaustive overview of the present disclosure. It is not intended to identify key or important parts of the disclosure, nor is it intended to limit the scope of the disclosure. Its purpose is merely to present some concepts in a simplified form as a prelude to the more detailed description that is discussed later.

One of the objectives of the present disclosure is to provide a fluid supply unit, a micro-droplet ejection driving device, and a generating device to overcome at least the above-mentioned problems in the prior art.

According to an aspect of the present disclosure, there is provided a micro-droplet ejection driving device including a housing in which a fluid supply unit can be accommodated, and a piezoelectric actuator fixed on the housing and configured to communicate with the fluid. An outer wall of the supply unit is in close contact and drives the outer wall to vibrate.

According to another aspect of the present disclosure, there is provided a fluid supply unit including: a fluid ejection portion and an energy conductive sheet, wherein the fluid ejection portion and the energy conductive sheet constitute at least part of a container wall of a container for the fluid to be ejected. The energy conductive sheet is used to be in close contact with the end surface of the piezoelectric actuator and is driven to generate vibration, so that the fluid is ejected through the fluid ejection portion to form a directional micro-droplet stream.

According to still another aspect of the present disclosure, there is provided a micro-droplet ejection generating device including a combination of any one of the forms of the micro-droplet ejection driving device and any of the forms of the fluid supply unit.

According to the fluid supply unit, the micro-droplet ejection driving device and the generating device of the present disclosure, the fluid ejection unit is provided on the fluid supply unit and is independent of the driving device, so that the fluid supply unit can be replaced independently. In the case of changing the fluid supply unit, the driving device can be used repeatedly. The fluid supply unit and the micro-droplet ejection driving device can cooperate with each other to form a micro-droplet ejection generating device. In use, the driving device does not contact the chemical liquid in the fluid supply unit, which can avoid cross-contamination.

These and other advantages of the present disclosure will be more apparent through the following detailed description of the preferred embodiments of the present disclosure in conjunction with the accompanying drawings.

### BRIEF DESCRIPTION OF THE DRAWINGS

The present disclosure may be better understood by referring to the description given below in conjunction with the accompanying drawings, wherein the same or similar reference numerals are used throughout the drawings to refer to the same or similar components. The drawings together with the following detailed description are included in and form a part of this specification, and are used to further illustrate preferred embodiments of the present disclosure and explain the principles and advantages of the present disclosure, wherein:
FIG. 1 is a schematic diagram of an embodiment of a micro-droplet ejection driving device of the present application;
FIG. 2 is a schematic diagram of an embodiment of a standing wave piezoelectric actuator of the present application;
FIG. 3 is a schematic diagram of another embodiment of a standing wave piezoelectric actuator of the present application;
FIG. 4 is a schematic diagram of an embodiment in which a guiding light source according to the present application is disposed on a housing;
FIG. 5 is a schematic diagram of an embodiment in which a shielding mechanism is provided on a housing of the present application;
FIG. 6 is a schematic diagram of a specific embodiment of a blocking mechanism;
FIG. 7 is a schematic diagram of another embodiment of a blocking mechanism, wherein the blocking mechanism is in a closed state;
FIG. 8 is a schematic diagram of another embodiment of a blocking mechanism, wherein the blocking mechanism is in an open state;
FIG. 9 is a schematic diagram of an embodiment of a fluid supply unit of the present application;
FIG. 10 is a schematic diagram of an embodiment of connecting a fluid supply unit and a fluid storage unit by using an interface;.
FIG. 11 is a schematic structural diagram of a U-shaped groove provided in the fluid supply unit;
FIG. 12 is a schematic diagram of a positional relationship between a U-shaped groove and a fluid ejection portion;
FIG. 13 is a schematic diagram of an embodiment of the micro-droplet ejection generating device according to the present application;
FIG. 14 is a schematic diagram of another embodiment of a micro-droplet ejection generating device according to the present application;
FIG. 15 is a schematic diagram of a third embodiment of the micro-droplet ejection generating device according to the present application;
FIG. 16 is a schematic structural diagram of a standing wave piezoelectric actuator cooperating with a fluid supply unit in a small package;
FIG. 17 is a schematic diagram of ejecting eye drops on a target by using the micro-droplet ejection generating device of the present application;
FIG. 18 is a schematic diagram of a fourth embodiment of the micro-droplet ejection generating device of the present application;
FIG. 19 is a schematic diagram of an embodiment of a detachable connection between a fluid supply unit and a housing in the micro-droplet ejection generating device of the present application;
FIG. 20 is a schematic diagram of an embodiment in which a sensor is provided on the micro-droplet ejection generating device of the present application;
FIG. 21 is a schematic diagram of another embodiment in which a sensor is provided on the micro-droplet ejection generating device of the present application.

Those skilled in the art will understand that elements in the drawings are illustrated for simplicity and clarity and have not necessarily been drawn to scale. For example, the dimensions of some of the elements in the drawings may be exaggerated relative to other elements to help improve the understanding of the embodiments of the present invention.

### DETAILED DESCRIPTION

Hereinafter, exemplary embodiments of the present disclosure will be described with reference to the accompanying drawings. In the interest of clarity and conciseness, not all features of an actual implementation are described in the specification. However, it should be understood that in the development of any such practical embodiment, many implementation-specific decisions must be made in order to achieve the developer's specific goals, such as meeting those system and business-related constraints, and these restrictions may vary depending on the implementation. In addition, it should also be understood that, although development work may be very complex and time-consuming, it will only be a routine task for those skilled in the art having the benefit of this disclosure.

Here, it should be noted that in order to avoid obscuring the present disclosure due to unnecessary details, only the device structure and / or processing steps closely related to the solution according to the present disclosure are shown in the drawings, and other details not relevant to this disclosure are omitted.

The description below is in the following order:
1. Micro-droplet ejection driving device
2. Fluid supply unit
3. Micro-droplet ejection generating device

### 1. Micro-droplet ejection driving device

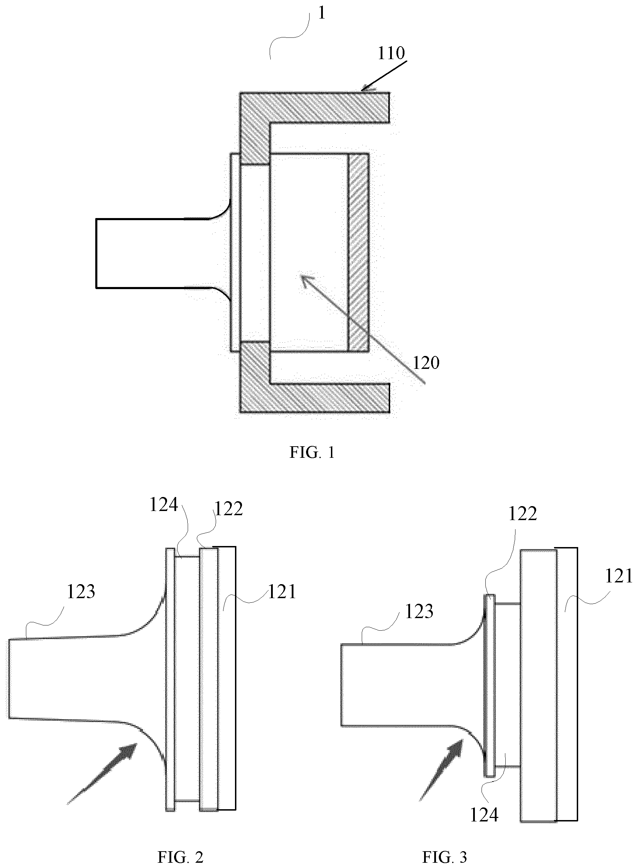

FIG. 1 is a schematic structural diagram of a micro-droplet ejection driving device 1 according to an embodiment of the present disclosure.

As shown in FIG. 1, the micro-droplet ejection driving device 1 according to the embodiment of the present disclosure may include a housing 110 and a piezoelectric actuator 120, and the piezoelectric actuator 120 is fixed on the housing 110.

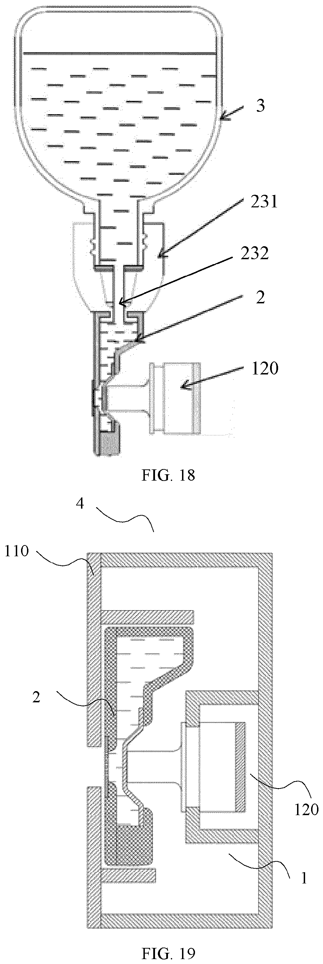

Furthermore, in combination with FIG. 19, the housing 110 can also be used to accommodate the fluid supply unit 2; the piezoelectric actuator 120 is also configured to be in close contact with an outer wall of the fluid supply unit 2 and drive the outer wall to vibrate.

As an example of the present disclosure, the micro-droplet ejection driving device 1 may exist independently to provide source power for liquid ejection to the fluid supply unit 2. In addition to providing an attachment space for the piezoelectric actuator 120, the housing 110 can also be used to connect the fluid supply unit 2. Thereby, the relative position of the piezoelectric actuator 120 and the fluid supply unit 2 is defined, so that the spatial relationship of close contact is achieved.

The vibration effect of the piezoelectric actuator 120 is related to its resonance frequency. In actual use, it can be selected according to requirements.

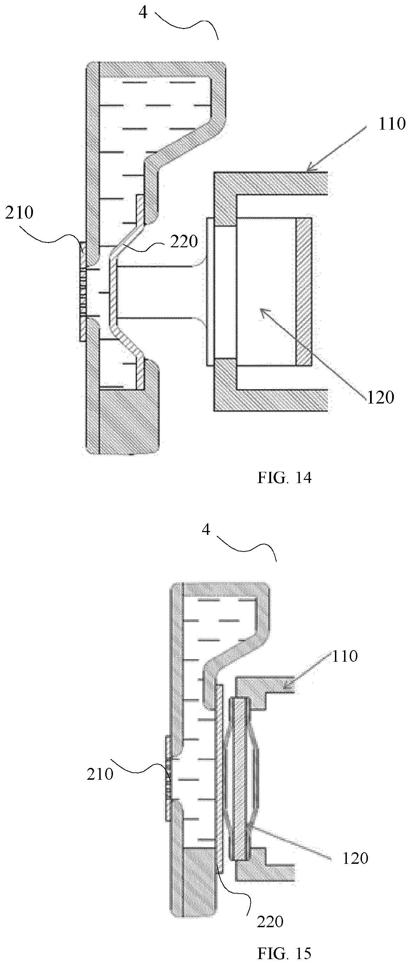

The piezoelectric actuator 120 may be a cymbal-liked piezo actuator. As shown in FIG. 15, it includes a piezo-type driving element in the middle and two cymbal-liked thin plates on both sides of the piezo driving element in structure. The convex surface of the cymbal-liked thin plate faces the outer end, and the bottom surface is connected with the piezoelectric driving element; the resonance frequency of the cymbal-liked piezoelectric actuator is related to the material, thickness and diameter of the piezo-type driving element, and it is also related to the material and the thickness, as well as the diameter of the base and the diameter of the bulge.

The piezoelectric actuator 120 may also be a standing wave piezoelectric actuator. A standing wave is a superimposed wave formed when two columns of coherent waves with the same amplitude travel in opposite directions on the same straight line. In order to meet the conditions of standing wave formation and obtain the maximum displacement output at the energy output end of the piezoelectric actuator, the entire length of the vibration part of the standing wave piezoelectric actuator must be half-wavelength of ultrasonic waves or integer multiple of half-wavelength of ultrasonic waves; the wavelength of the ultrasonic wave is related to the resonance frequency of the piezoelectric drive element. In order to make the piezoelectric drive element and the excitation source produce mechanical resonance to obtain the maximum displacement output, the frequency of the excitation source needs to be the same as the resonance frequency of the piezoelectric driving element to generate resonance. Since the entire length of the vibrating part includes at least two quarter wavelengths, and the length of the quarter wavelength is the distance between the maximum amplitude point and the adjacent node, the node can be used as a connection fixed point.

According to a preferred embodiment of the present disclosure, in combination with the standing-wave piezoelectric actuator shown in FIG. 2 and FIG. 3, the structure includes a piezoelectric driving element 121 and an energy horn. The shaft segment 122 and the cylindrical segment 123 are an integral part. The piezoelectric driving element 121 is connected to one side of the disc segment 122. The output end of the cylindrical segment 123 is used for tight contact with the fluid supply unit 2.

Further, as shown in FIG. 2, the disc segment 122 may be provided with a circular groove 124 on the side close to the cylindrical segment 123, and the piezoelectric actuator 120 and the housing 110 may be connected through the circular groove 124.

As shown in FIG. 3, the disc segment 122 can also be set as a large diameter segment and a small diameter segment, and the large diameter segment can be on the side close to the piezoelectric driving element 121; then a circular groove 124 can be provided on the small diameter segment. It is a structure which realizes connection with the housing 110. The large-diameter section can be selected to have the same cross-sectional area as the piezoelectric driving element 121 and is used to transmit the driving force of the piezoelectric driving element 121. The small-diameter section can play a role of energy gathering.

According to the embodiment of the present disclosure, the housing 110 may be connected to the piezoelectric actuator 120 through a clamping mechanism inside the housing 110, for example, the annular groove 124 is selected as a clamping point.

In theory, if the clamping mechanism is connected to the vibration node of the energy horn, the smaller the influence on the vibration effect of the piezoelectric actuator 120 is, the harder it is to achieve this ideal state in actual use. Therefore, as shown in FIG. 2 and FIG. 3, the clamping point of the clamping mechanism can be selected to be located at a near node on the side of the disc segment 122 near the cylindrical segment 123.

In order to maximize the displacement output from the vibration output end of the standing wave piezoelectric actuator, so that the fluid in the fluid supply unit 2 obtains a greater displacement force, theoretically, the length sum of the piezoelectric driving element 121 and the energy horn can be selected to be an ultrasonic half-wavelength or an integer multiple of the ultrasonic half-wavelength. For example, the length and length of the piezoelectric driving element 121 and the disk segment 122 can be set to a quarter ultrasonic wavelength or n times the wavelength of the quarter ultrasonic wave, and the length of the cylindrical segment 123 is set to three quarters of the ultrasonic wavelength or n times of the three quarters of the ultrasonic wavelength, where n is a positive integer. This is only a theoretical value that is considered in terms of effects. In practical applications, it can be chosen according to actual needs.

According to the embodiment of the present disclosure, in order to achieve better energy gathering and driving effect, the length of the disc segment 122 of the energy horn can be selected from a range of 1mm to 10mm, and the diameter can be selected from a range of 5mm to 20mm; The length of the segment 123 can be selected in the range of 5mm to 15mm, and the diameter can be selected in the range of 2mm to 6mm; the corresponding excitation source frequency is selected between 50KHz-200KHz. For the actual combination of the disk segment 122 and the cylindrical segment 123, the constraint conditions that can maximize the output displacement of the piezoelectric actuator can be selected; and the frequency of the excitation source can be selected after comprehensively considering the resonance frequency of the piezoelectric driving element 121 and the design size of the energy horn.

In order to reduce the energy loss in the vibration transmission process and improve the resonant frequency operating bandwidth of the piezoelectric actuator 120, the abrupt cross-section between the disc section 122 and the cylindrical section 123 may transition into a concave rounded portion. For example, the cross-sectional area of one end of the cylindrical segment 123 is gradually increased until it joins the disc segment 122, so as to form a chamfered transition from the cylindrical segment 123 to the disc segment 122.

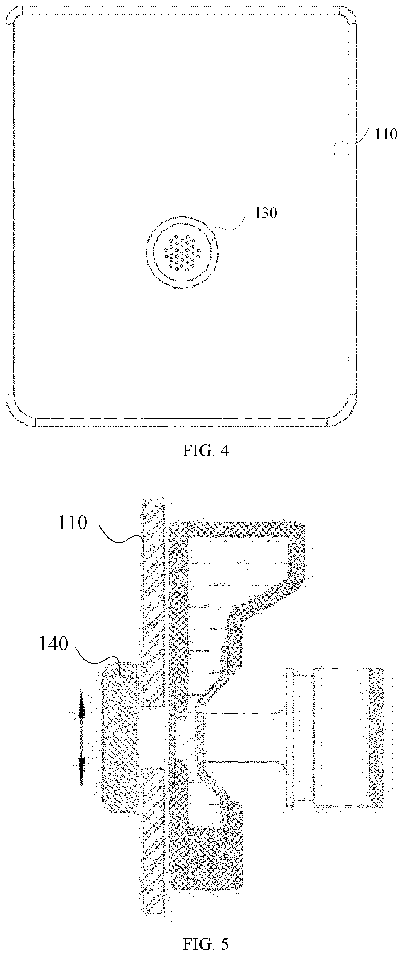

According to the embodiment of the present disclosure, as shown in FIG. 4, a guiding light source 130 may be further provided to help the ejection target to position itself relative to the ejection device.

The guiding light source 130 may be optionally disposed on the housing 110, for example, on the front cover of the housing 110, and may be further disposed at a position corresponding to the fluid ejection portion of the fluid supply unit 2, such as a peripheral area of the fluid ejection portion.

The guide light source 130 may also be selected and arranged on other structures that can guide the ejection target for positioning, as long as the person being injected can perceive the position of the specific ejection port. So as far as possible to align the ejection target and the ejection port, the design goal is achieved.

FIG. 4 as an example, the guiding light source 130 may be configured as an annulus light belt, connected to the front cover of the housing 110, and may surround a peripheral position corresponding to the fluid ejection portion.

Keeping the drug delivery device and the ejection target in the correct relative position is the key to better ejection administration. For example, when administering to the eye, the ejection port of the administration device should face the eye. In order to prevent damage to the eyes, the guiding light source 130 of the present disclosure may use a soft, non-irritating and non-harmful light source to effectively assist in positioning. The guiding light source 130 is particularly suitable for positioning when other people assist in drug administration, for example, by judging through the light reflected from the eyeball of the user to determine an appropriate ejection position.

For the self-use eye ejection operation, in addition to the guidance of the guiding light source 130, a suitable position can be found as soon as possible by the user's practice. For example, for ejection of eye drops, the ejection device needs to be about 2 to 5 cm from the eye. After several uses, the user can find a more suitable ejection position by relying on the relative position of the palm and the face.

In order to prevent the fluid supply unit 2 from being polluted in various forms due to exposure to the air after use, according to an embodiment of the present disclosure, as shown in FIG. 5, a shielding mechanism 140 may be further provided on the housing 110. 1 For example, the structure of the housing 110 may include a front cover, which may be located in front of the fluid ejection portion of the fluid supply unit 2 and open a hole at a position corresponding to the fluid ejection portion. The shielding mechanism 140 may adopt a design form capable of opening or closing the through hole, opening or closing the fluid ejection portion of the fluid supply unit 2 to ensure that the device is isolated from the external environment when the device is not in use.

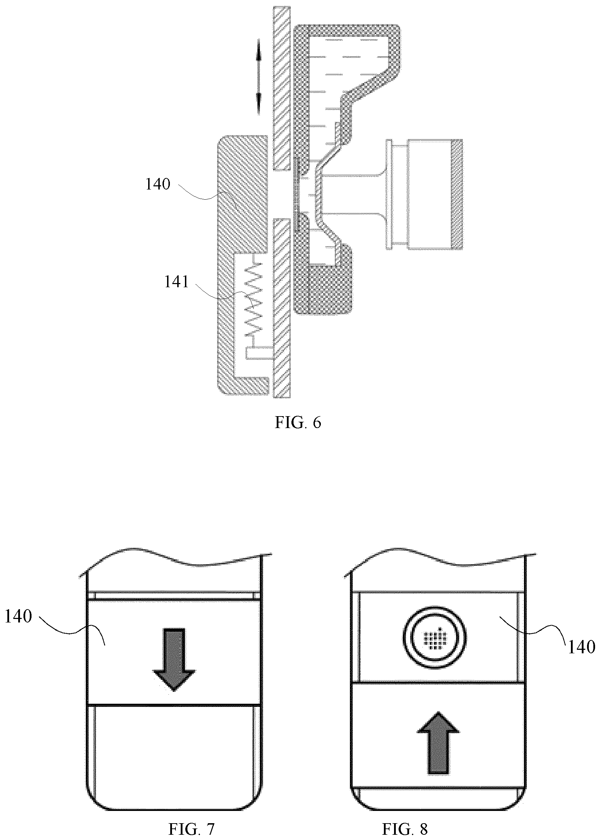

Further, as shown in FIG. 6, the specific implementation form of the shielding mechanism 140 may be an outer cover including an elastic structure 141. When the elastic structure 141 is selected as a spring, one end of the spring may be fixed on the housing 110 through a connecting member. The other end is connected to the cover. Under normal conditions, the spring is in an extended state, and the outer cover just closes the fluid ejection portion.

Pressing the outer cover to compress the spring, exposing the through hole of the housing 110, and the fluid ejection portion can be opened; the spring can naturally return to its original state after being pressed by the external force, and then, closing the fluid ejecting part to protect the micro-nozzle sheet from contamination.

In specific implementation, the function of the blocking mechanism 140 does not necessarily have to be achieved by expansion of the spring, but can also be achieved by an elastic torsion structure.

In addition, in combination with FIG. 7 and FIG. 8, the blocking mechanism 140 may also be implemented by a sliding cover. For example, a slide groove may be provided on the housing 110 so that the slide cover can slide up and down along the slide groove, thereby opening or closing the fluid ejection portion. FIG. 7 is a schematic diagram of the sliding cover covering the fluid ejecting portion; when in use, the sliding cover is lowered, as shown in FIG. 8, which is a schematic diagram of opening the fluid ejecting portion.

The sliding cover can also be used as a basis for judging whether the ejection is wrong. If the sliding cover is closed, the sensor can transmit a closing signal to the corresponding controller, and the controller turns off the function of the ejection switch accordingly, thereby preventing the waste caused by misuse and polluting the inside of the device.

### 2. Fluid supply unit

According to an embodiment of the present disclosure, a fluid supply unit 2 is also provided.

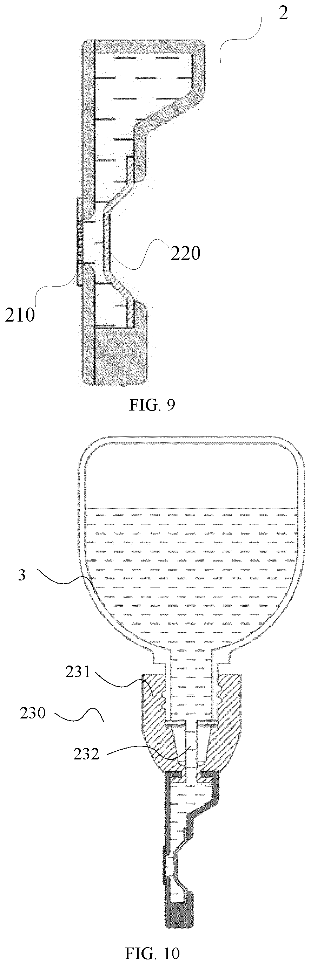

As shown in FIG. 9, the fluid supply unit 2 includes: a fluid ejection portion 210 and an energy conductive sheet 220, wherein the fluid ejecting portion 210 and the energy conducting sheet 220 constitute at least part of the container wall of the container to be ejected; and the energy conducting sheet 220 is used to be in close contact with the end surface of the piezoelectric actuator 120 and is driven to generate vibration, so that the fluid is ejected through the fluid ejection section 210 to form a directional droplet stream.

According to the present disclosure, a fluid is contained in the fluid supply unit 2 through a container, and the energy conductive sheet 220 on the wall of the container is used to receive vibration energy of the piezoelectric actuator 120 to generate vibration, thereby pushing the internal fluid through the fluid ejection portion 210 to form a micro-droplet stream.

The fluid supply unit 2 can be used as an independent structure, which is convenient to carry and can be replaced as needed. The fluid supply unit 2 can be set into small packages according to the dosage, so that the medicinal solution inside thereof is used up within a proper use period, so that the medicinal solution without adding a preservative can be used economically and effectively without causing waste.

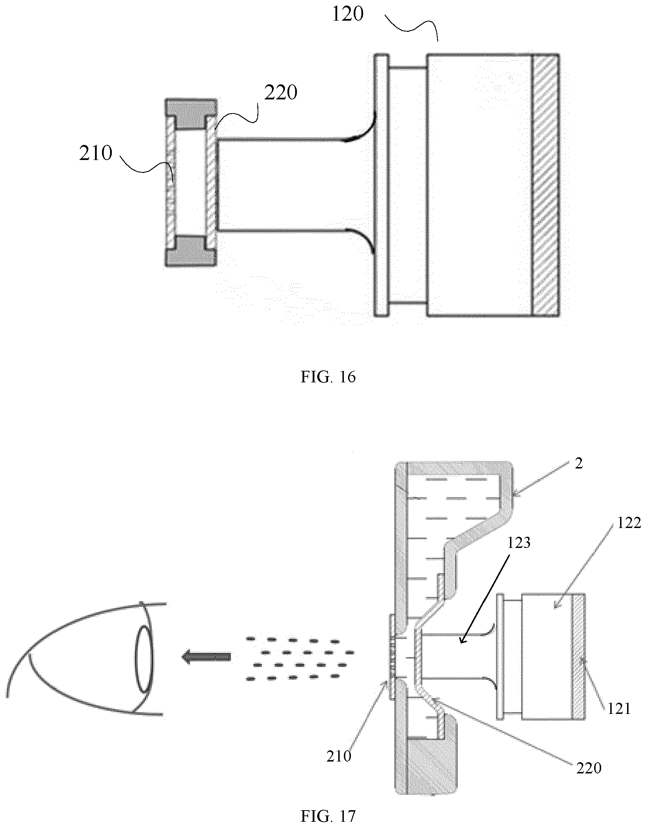

The fluid in the fluid supply unit 2 such as a medicinal solution can be filled in a qualified pharmaceutical factory and used in conjunction with the micro-droplet ejection driving device 1, as shown in FIG. 17, which is a schematic diagram of an eye drops ejection.

The dose parameters of the contents of the fluid supply unit 2 can be adjusted and set according to individual medicaments, and a uniform dose concentration can also be set. The capacity of the fluid supply unit 2 can be large, such as a dropper on the market; it can also be small to meet the need for no preservatives.

According to a preferred embodiment of the present disclosure, the fluid supply unit 2 may further include an interface 230. For example, for the fluid supply unit 2 provided in a small package, when the internal fluid is used up, as shown in FIG. 10, the fluid supply unit 2 may be communicated with the fluid storage unit 3 configured through the interface 230 to supplement the supply.

Further, as shown in FIG. 10, the specific structure of the interface 230 may include a connection urging member 231. The function of the connection urging member 231 is to enable the container for the fluid to be ejected to form a closed connection with the fluid storage unit 3. Without causing the fluid to come into contact with the outside air during the flow process, and cause the property to change.

The interface 230 may further include a piercing portion 232, which can be used to pierce the closed outlet of the fluid storage unit 3 so that the fluid inside can enter the container for the fluid to be ejected. The piercing part 232 may adopt a siphon and breathable structure (not shown), so that fluid can smoothly flow out to the fluid supply unit through the piercing part 232.

According to the embodiment of the present disclosure, the connection pressing member 231 and the fluid storage unit 3 may be a snap-type connection; a threaded structure may also be used for connection.

According to a preferred embodiment of the present disclosure, in order to achieve a better ejecting effect, it is necessary to reduce the loss of driving energy inside the container, and therefore, a relatively short transmission path may be selected to set the fluid ejecting portion 210 and the energy conductive sheet 220. For example, the two of them are placed directly opposite to each other on the container wall of the fluid to be ejected, as shown in FIG. 9.

In fact, if the fluid ejection portion 210 and the energy transmission sheet 220 are disposed at other positions, and fluid ejection can also be achieved. For example, the two of them are arranged on adjacent surfaces of the container wall, and when the energy conducting sheet 220 is pressed by the fluid inside the container under vibration, the fluid can also be ejected.

According to the present disclosure, the fluid ejection portion 210 may be implemented by using a micro-nozzle sheet, as shown in FIG. 9. The micro-nozzle sheet can be made of one of the following materials: metal, polymer material, ceramic material or single crystal material. Metal materials include, but are not limited to, stainless steel, nickel, nickel-cobalt alloys, etc.; polymer materials, such as polyetheretherketone (PEEK); ceramic materials, such as alumina, zirconia, etc.; single crystal materials, such as sapphire, silicon carbide, etc.

At least one nozzle hole must be provided on the micro-nozzle sheet to ejection fluid.

The number of nozzle holes can be determined by comprehensive consideration in combination with the size of the ejection target site, the desired ejection effect and the source driving force. For a micro-nozzle sheet with multiple nozzle holes, multiple nozzle holes can adopt various regular or irregular arrangements. For example, the plurality of nozzle holes may be in the form of an equidistant arrangement of honeycombs.

According to a preferred embodiment of the present disclosure, for the arrangement form of multiple nozzle holes, in order to make the directional micro-droplet stream ejected relatively concentrated within a certain range and produce a better ejection effect for the ejection target, nozzle holes can be distributed in the area of 1 mm to 10 mm in diameter with the center as the origin on the micro-nozzle sheet. The diameter of the nozzle hole can be selected from 5 to 200 microns..

By selecting the number of nozzle holes and the diameter of the nozzle holes, the amount of fluid ejected by the micro-nozzle sheet at a time can range from 1 microliter to 200 microliters. For the case where the fluid is an eye drop, the numerical range is verified by experiments to be able to meet the effective drug dose required for the eyeball after the loss of drug is discounted.

The preferred amount of fluid ejected by the micro-nozzle hole per time is 8 microliters to 15 microliters.

A further preferable value of the amount of fluid ejected by the micro-nozzle hole per time is 10 microliters.

In order to better generate the directional micro-droplet stream, the structure of the nozzle hole should satisfy that a laminar flow can be generated when the fluid passes, and the reverse air flow can be reduced in the process of generating the directional micro-droplet stream. In order to meet this requirement, the shape of the nozzle hole can be set to gradually change the diameter from the inner wall side to the outer wall side of the container, for example, the diameter of the fluid inlet end of the nozzle hole is larger than the diameter of the fluid outlet end. Further, the shape of the nozzle hole can be set to be tapered, and its side wall can be bent toward the center of the hole to form an arc-shaped wall.

In addition, as an alternative, the guiding light source 130 provided on the housing 110 of the micro-droplet ejection driving device 1 may also be provided on the micro-nozzle hole; and further, the guiding light source 130 may be formed into a halo and disposed at the periphery of the nozzle hole area.

According to the embodiment of the present disclosure, in order to improve the ejecting efficiency of the fluid and make it less residue on the container wall, a hydrophobic layer may be provided on the sidewall surface of the nozzle hole; or to minimize the outflow of the fluid from the nozzle hole in a non-jetting state, a hydrophilic layer may be provided on the sidewall surface of the nozzle hole. Both the hydrophobic layer and the hydrophilic layer are provided to make the micro-nozzle sheet have good biocompatibility.

Further, the hydrophobic layer or the hydrophilic layer may be a newly added coating. For example, additional coatings using metal, cermet or ceramic materials are used. Among them, metals such as gold, palladium, platinum, titanium, tin-cobalt alloys, etc.; cermets such as chromium carbide.

The hydrophobic layer or the hydrophilic layer can also be obtained by material modification of the sidewall surface of the nozzle hole.

According to a preferred embodiment of the present disclosure, for example, the material of the micro-nozzle sheet can be selected from a nickel-cobalt alloy, and a chromium carbide or gold coating can be added to the sidewall surface of the nozzle hole, thereby obtaining a hydrophilic surface with smooth surface and good biocompatibility.

Or, in order to increase the hydrophobicity of the surface of the nozzle hole, a surface of the side wall of the nozzle hole may be coated with, for example, a Teflon coating.

Material modification of the surface of the side wall of the nozzle hole can be used to nanometerize the surface structure of the nozzle hole, and can also achieve the purpose of hydrophobicity or hydrophilicity.

For example, an aluminum material can be used to make a micro-nozzle sheet, and the surface of the side wall of the nozzle can be anodized to obtain a thin layer of alumina to form a hydrophilic coating.

The nozzle holes can be obtained by one of the following machining methods: for example, drilling, punching, or shearing.

Or the nozzle holes can be obtained by one of the following non-mechanical processing methods: electroforming, etching and other electrochemical methods; laser burning or ablation.

According to the embodiment of the present disclosure, in order to ensure the safe use performance of the fluid inside the fluid supply unit 2, it is necessary to isolate it from the external space. That is to form a closed space within the nozzle hole.

For example, a removable surface layer may be provided on the nozzle holes, and the surface layer covers the outer surface of the micro-nozzle sheet to seal all the nozzle holes. When using, just remove it.

Alternatively, a sealing film is provided for sealing each nozzle hole separately; the sealing film is broken when the fluid in the container is pressurized.

Alternatively, a layer of sealing wall may be provided in the container for the fluid to be ejected, and the inner cavity of the container may be divided into a fluid storage area near the piezoelectric actuator and an ejection area near the fluid ejection portion. In the initial state, the fluid is stored in the fluid storage area, and when the fluid is pressurized, the sealing wall is pushed to rupture.

According to the embodiment of the present disclosure, the shape of the energy conducting sheet 220 may be various, such as flat, concave, convex, or curved surface, etc., which can meet the requirements for use in different situations, respectively.

For example, the energy conducting sheet 220 may be selected to have a flat plate shape, as shown in FIGS. 15 and 16. The flat plate-shaped energy conducting sheet 220 can be used in combination with a piezo-type piezoelectric actuator, as shown in FIG. 15; it can also be used in combination with a standing-wave piezoelectric actuator, as shown in FIG. 16.

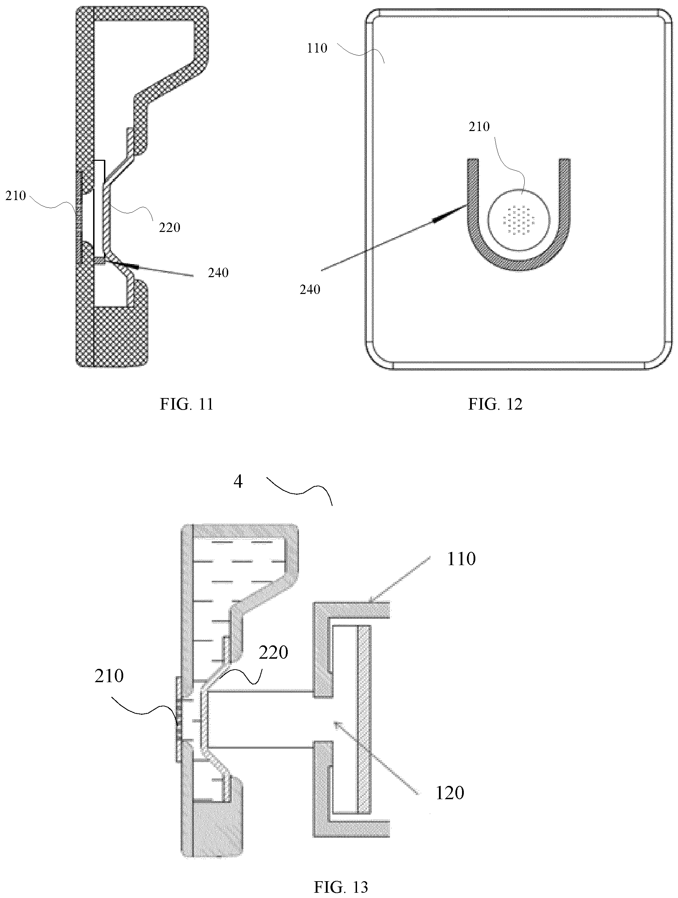

The shape of the energy conducting sheet 220 may also be a shape recessed toward the fluid side, and the bottom surface of the recess is configured to be in close contact with the end surface of the piezoelectric actuator to receive vibration energy. Can be used in conjunction with standing wave piezoelectric actuators, as shown in Figures 13 and 14.

In order to make the energy conducting sheet 220 have sufficient strength and to minimize losses in the energy transmission process, a metal sheet having a thickness of 0.01 mm to 0.2 mm can be used.

According to a preferred embodiment of the present disclosure, the energy conducting sheet 220 may be made of a 316L stainless steel material with a thickness of 0.05 mm.

Alternatively, the energy conducting sheet 220 may be made of a non-metal film having a thickness of 0.01 mm to 0.1 mm.

Preferably, the energy conducting sheet 220 may be made of a 0.04 mm thick natural latex film.

According to the embodiment of the present disclosure, as shown in FIG. 11 and FIG. 12, the fluid supply unit may further include a U-shaped clamping groove 240, and the U-shaped clamping groove 240 may be disposed on an inner wall surface of the container to be ejected, and It is located at the periphery of the fluid ejection portion and is used to provide a storage space for a small volume of fluid. When the fluid supply inside the container to be ejected is insufficient and only a small amount is left, a small amount of fluid can stay in the U-shaped groove 240 due to surface tension by shaking, so as to be close to the fluid ejection portion 210, which is beneficial to improve ejection utilization while reducing residual waste.

### 3. Micro-droplet ejection generating device

Another embodiment of the present application provides a micro-droplet ejection generating device 4.

With reference to FIG. 13, FIG. 14, and FIG. 15, the micro-droplet ejection generating device 4 may be a combination of any specific embodiment of the micro-droplet ejection driving device 1 and any specific embodiment of the fluid supply unit 2. After the combination, a complete fluid ejection device is formed.

The micro-droplet ejection driving device 1 and the fluid supply unit 2 can be detachably connected to facilitate the replacement of the fluid supply unit 2 and to restrict the relative positional relationship between the piezoelectric actuator 120 and the energy conductive sheet 220. Therefore, they can contact each other and better transmit vibration energy.

According to the embodiment of the present disclosure, in combination with FIG. 15, a fluid supply unit 2 having a flat plate-shaped energy conductive sheet 220 may be combined with a micro-droplet ejection driving device 1 including a piezo-type piezoelectric actuator to form a micro- droplet ejection generating device 4. The convex surface of a thin-type thin plate of a single-type piezoelectric actuator serves as a driving end and is in close contact with the energy conducting sheet 220.

According to still another embodiment of the present disclosure, as shown in FIG. 13, an example in which the fluid supply unit 2 is combined with a micro-droplet ejection driving device 1 including a standing-wave piezoelectric actuator, the end of a cylindrical segment 123 of the standing wave piezoelectric actuator can be used as the output end to closely contact the energy conducting sheet 220. The shape of the energy conducting sheet 220 may be recessed toward the fluid side, and the size of the recessed portion may just match the output end of the cylindrical section 123. An annular groove 124 may be provided on the cylindrical section 123. The annular groove 124 is located near the disc section 122. The clamping mechanism of the housing 110 may be connected to the annular groove 124.

According to yet another embodiment of the present disclosure, the micro-droplet ejection generating device 4 shown in FIG. 14 is different from FIG. 13 in that the annular groove 124 is provided on the disc section 122 and is located near the cylindrical section 123. In position, the housing 110 may be connected to the annular groove 124 by a clamping mechanism.

FIG. 16 shows an example in which a micro-droplet ejection driving device 1 including a standing-wave piezoelectric actuator is combined with a small-package fluid supply unit 2. Since the inner volume of the fluid supply unit 2 is relatively small, a flat plate-shaped energy conductive sheet 220 may be selected. In order to reduce the waste of fluid in a small space and minimize the dead angle of the fluid ejection inside, it is possible to choose to make the shape and size of the fluid ejection part 210 and the energy conducting sheet 220 the same, and to form almost the entire length of the container wall of the fluid supply unit 2 while keeping the two directly opposite; in this way, the action surface of the driving force and the content is increased, and the residue can be reduced.

In order to achieve a better ejecting effect, the micro-nozzle sheet, the energy conductive sheet 220 and the piezoelectric actuator 120 may be coaxially arranged, such as the corresponding structures shown in FIGS. 13 to 17. The coaxial arrangement can make the driving force more directly transmitted to the fluid, so that the fluid can be ejected from the nozzle after a relatively short path.

The distance between the micro-nozzle hole and the energy-conducting plate 220 will also affect the energy transfer. When the distance is too close, fluid replenishment rate may be insufficient, which will affect the ejecting effect. At the same time, if the volume formed between the small distance is too small, it is not suitable for the supply of larger volumes; if the distance is too large, it will increase the energy loss and affect the ejection effect.

According to the embodiment of the present disclosure, the distance between the micro-nozzle sheet and the energy conducting sheet 220 may be selected within a range of 0 to 3 mm according to actual use needs. The selection of the distance is related to the magnitude of the driving force, the desired ejection speed, the diameter of the ejected droplets, and the amount of deposition on the target. As the driving voltage increases, the distance between the two can be appropriately increased.

A preferred distance between the micro-nozzle sheet and the energy conducting sheet 220 is 0.2-0.8 mm.

FIG. 17 is a schematic diagram of the micro-droplet ejection generating device 4 ejecting eye drops on a target when the micro-nozzle sheet, the energy conductive sheet 220 and the piezoelectric actuator 120 are coaxially arranged. It can be seen from FIG. 17 that the droplet stream is basically distributed in a range facing the eyeball, which is beneficial to obtain a good ejecting effect.

According to the embodiments of the present disclosure, by selecting a driving voltage and a nozzle size, a droplet with a target initial speed and size can be generated. For example, the driving voltage is selected so that the initial velocity of the micro-droplet stream generated by the fluid ejection section 210 is in the range of 0.5 m/s to 20 m/s; and the combination of the selection of the nozzle size makes the droplet diameter greater than 15 microns; in combination with the adjustment of the ejection target position, at least 70% of the micro-droplet stream can be deposited on the target.

Alternatively, the energy conducting sheet 220 and the piezoelectric actuator 120 can be in close contact, and the nozzle holes are designed to be arranged in an equal distance honeycomb. By selecting specific parameters, micro droplets with a target speed of 0.5 m/s to 10 m/s and a diameter of 10 to 100 microns can be generated..

According to yet another specific embodiment of the present disclosure, the diameter of the nozzle hole can be selected to be 35 micrometers, so that the initial average ejection velocity of the fluid is 10 m/s, and the ejecting distance is 20 mm, thereby achieving a deposition ratio of 70% of the fluid on the ejection target..

According to still another specific embodiment of the present disclosure, for the case where the polyvinyl alcohol fluid is contained in the fluid supply unit 2, the micro-nozzle sheet, the energy conducting sheet 220, and the piezoelectric actuator 120 may be coaxially arranged to make the distance between the micro-nozzle hole sheet and the energy conducting sheet 220 is 0.2-0.3mm; a micro-perforated sheet made of nickel-containing material is selected, and a tin-cobalt alloy coating can be selected as the coating on the surface of the perforated sheet to prevent the interior of the micro-perforated sheet material Nickel precipitates, causing the droplets to be contaminated.

FIG. 18 shows still another specific embodiment of the micro-droplet ejection generating device 4. A standing-wave piezoelectric actuator is used to cooperate with the energy conducting sheet 220 having a concave shape; the container for the fluid to be ejected is connected to the fluid storage unit 3 through the connection pressing member 231, and the connection method may be a snap-on type or a screw type; the fluid storage unit 3 is punctured by the puncture portion 232, so that the fluid storage unit 3 is communicated with the container.

An embodiment of the application interface 230, for example, the fluid supply unit 2 contains eye drops, and the fluid supply unit 2 is combined with a commercially available eye dropper through the interface 230. As shown in FIG. 18, an opening may be provided above the fluid supply unit 2. A connection pressing member 231 made of silicone is combined with the opening, and the puncture part 232 may be connected to the connection pressing member 231. After the eyedrops dropper is opened, the bottle cap is opened, and then the liquid ejection head is directly connected to the pressing member 231. Under the guidance of the connecting pressing member 231, the puncture portion 232 penetrates the liquid ejection head. After expanding the holes, the two are communicated, and the medicinal solution is introduced into the fluid supply unit 2.

FIG. 19 shows another specific embodiment of the micro-droplet ejection generating device 4. The housing 110 is provided with a structure for installing the fluid supply unit 2, which can realize the detachable connection of the fluid supply unit 2, and can also make the fluid supply unit 2 is in close contact with the end of the piezoelectric actuator 120; the position of the fluid ejection portion 210 corresponds to a reserved opening in the housing 110; the clamping mechanism on the housing 110 is connected to the disc segment 122.

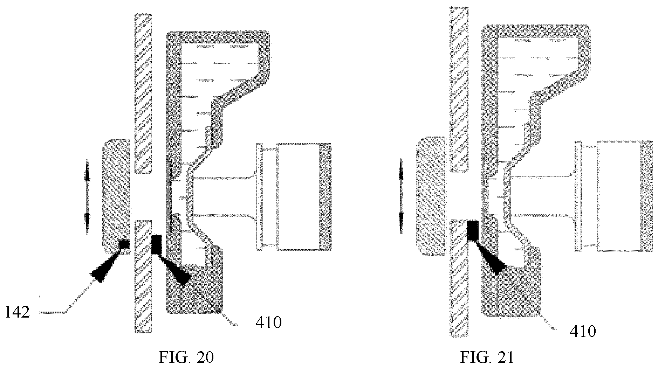

According to the embodiment of the present disclosure, as shown in FIG. 20 and FIG. 21, the micro-droplet ejection generating device 4 may further include a sensor 410, which detects whether the blocking mechanism 140 is opened by the sensor 410, and then transmits a detection signal to the controller; if the controller receives the opening signal of the blocking mechanism 140, when the liquid ejection switch is pressed, the controller controls the piezoelectric actuator 120 to be energized to generate vibration to perform liquid ejection; if the controller receives the closing signal of the blocking mechanism 140, then the controller outputs a control signal that prevents the piezoelectric actuator 120 from being energized. Even if the liquid ejection switch is pressed, ejection cannot be started, which can effectively prevent accidental ejection.

The sensor 410 may be disposed on the inner wall of the housing 110 and near the edge of the opening reserved on the housing 110.

Since the device provided in present disclosure is used in an externally bright state, the sensor 410 may be implemented using a photosensitive element such as a photoresistor, as shown in FIG. 21.

The sensor 410 may also be implemented by a Hall element. A magnet 142 may be provided on the blocking mechanism 140 to cooperate with the Hall element to determine the position of the blocking mechanism 140 and determine whether the blocking mechanism 140 is opened. The setting position of the magnet 142 may be: corresponding to the position of the Hall element when the blocking mechanism 140 is closed.

The following is a market survey of ejection doses: take eyedrops as an example, and investigate five commercially available eyedrops products. The data is shown in the table below.

It is understood that the proportion of eye drops is equivalent to water. According to the data in the table, the drop volume of a commercially available eye dropper is about 31 to 37 microliters. During use, about 50% to 90% of the liquid medicine will be lost, that is, only about 3.1 to 18.5 microliters of liquid medicine will enter the eyes. Some investigations indicate that the medicinal solution that the eyeball can hold is about 7 microliters, and this value is between 3.1 and 18.5 microliters. Therefore, it is a reasonable estimate to set a preferred effective dose of at least 7 microliters per time. In addition, considering that the effective droplet deposition amount that the micro-droplet ejection generating device of the present disclosure can achieve is at least 70%, if the ingredients of commercially available eye drops are used, the present disclosure should be capable of ejecting a dose of about 10 microliters at a time. In order to comply with the administration dose of the existing eye dropper, that is, one ejection is approximately equal to one drop.

In the case where the active ingredient of the potion is concentrated, the dose per ejection can be smaller.

**TABLE**

| No. | Eye Drop Brand | Specifications (ml) | Suggested dose | Aperture diameter (mm) | Average weight (mg) |
|---|---|---|---|---|---|
| 1 | New Rohto | 13 | 1-2 drops | 1.80 | 31.3 |
| 2 | Run Jie | 10 | 1-2 drops | 1.90 | 36.4 |
| 3 | Shanliang | 10 | 1-2 drops | 2.05 | 32.3 |
| 4 | ZhenShiMing | 15 | 1-2 drops | 2.10 | 36.2 |
| 5 | Ruizhu | 0.8 | 1 drop | 2.50 | 33.7 |

The technical solution of the present disclosure can be applied not only to the administration of eye drops in the eyes, but also to the precise administration of the oral cavity, the nasal cavity, the ears, the skin and other parts of the body.

In summary, according to the embodiments of the present invention, the present invention provides the following technical solutions, but is not limited thereto:
Technical Solution 1: A micro-droplet ejection driving device includes:
   A housing capable of containing a fluid supply unit;
      The piezoelectric actuator is fixed on the housing and is configured to be in close contact with an outer wall of the fluid supply unit and drive the outer wall to vibrate.
Technical solution 2: The micro-droplet ejection driving device according to technical solution 1, wherein the piezoelectric actuator is a standing wave piezoelectric actuator.
Technical solution 3: The micro-droplet ejection driving device according to technical solution 1, wherein the piezoelectric actuator is a cymbal-liked piezoelectric actuator.
Technical solution 4: The micro-droplet ejection driving device according to technical solution 2,
   The standing-wave piezoelectric actuator includes a piezoelectric driving element and an energy horn. The energy horn includes a coaxial disc segment and a cylindrical segment. The end face is used for close contact with the fluid supply unit.
Technical solution 5: The micro-droplet ejection driving device according to technical solution 4,
   The cylindrical segment and the disc segment transition with a concave rounded portion.
Technical solution 6: The micro-droplet ejection driving device according to any one of technical solutions 1 to 5, further comprising:
   The guiding light source is used for guiding and positioning the ejection target.
Technical solution 7: The micro-droplet ejection driving device according to any one of technical solutions 1 to 6, further comprising:
   A shielding mechanism provided on the housing is used to open or close a fluid ejection portion of the fluid supply unit.
Technical solution 8: The micro-droplet ejection driving device according to technical solution 7,
   The shielding mechanism is an outer cover including an elastic structure, which uses the elasticity of the elastic structure to open or close the fluid ejection portion.
Technical solution 9: A fluid supply unit, including:
   Fluid ejection section and energy conducting sheet,
   Wherein, the fluid ejection portion and the energy conducting sheet constitute at least part of a container wall of a container to which fluid is to be ejected; and
   The energy conducting sheet is in close contact with the end surface of the piezoelectric actuator and is driven to generate vibration, so that the fluid is ejected through the fluid ejection portion to form a directional micro-droplet stream.
Technical solution 10: The fluid supply unit according to technical solution 9, further comprising:
   Interface for communicating with a fluid storage unit.
Technical solution 11: The fluid supply unit according to technical solution 10,
   The interface includes a connection pressing member for guiding the fluid storage unit to form a tight connection with the container for the fluid to be ejected.
Technical solution 12: The fluid supply unit according to technical solution 11,
   The interface also includes a puncture portion for puncturing the fluid storage unit for communication.
Technical solution 13: The fluid supply unit according to any one of technical solutions 9 to 12, wherein the fluid ejection portion and the energy conductive sheet are oppositely disposed on the container for the fluid to be ejected.
Technical solution 14: The fluid supply unit according to any one of technical solutions 9 to 13, wherein the fluid ejection portion is a micro-nozzle sheet, and the micro-nozzle sheet has at least one nozzle hole.
Technical solution 15: The fluid supply unit according to technical solution 14, the nozzle holes are distributed in a region of 1 mm to 10 mm in diameter from the center of the micro-nozzle sheet, and the diameter of the nozzle holes is 5 µm to 200 µm.
Technical solution 16: The fluid supply unit according to technical solution 14 or 15, wherein a diameter of a fluid inlet end of the nozzle hole is larger than a diameter of a fluid outlet end.
Technical solution 17. The fluid supply unit according to technical solution 16, wherein the nozzle hole is a tapered hole, and a side wall thereof is bent toward the center side of the hole to form an arc-shaped wall.
Technical solution 18: The fluid supply unit according to any one of technical solutions 14 to 17,
   The sidewall surface of the nozzle hole has a hydrophobic layer or a hydrophilic layer.
Technical solution 19: The fluid supply unit according to technical solution 18,
   The hydrophobic layer or the hydrophilic layer is obtained by modifying the surface of the side wall of the nozzle hole.
Technical solution 20: The fluid supply unit according to any one of technical solutions 9 to 19,
   The utility model further comprises a U-shaped clamping groove, which is arranged on the inner wall surface of the container for the fluid to be ejected, and is located at the periphery of the fluid ejecting portion, and is used to provide a storage space for a small volume of fluid.
Technical solution 21, The fluid supply unit according to any one of technical solutions 9 to 20,
   The energy conducting sheet is made of a metal sheet having a thickness of 0.01 mm to 0.2 mm.
Technical solution 22: The fluid supply unit according to any one of technical solutions 9 to 20,
   The energy conducting sheet is made of a non-metal film with a thickness of 0.01 mm to 0.1 mm.
Technical solution 23: A micro-droplet ejection generating device includes the micro-droplet ejection driving device according to any one of Technical solutions 1 to 8 and the fluid supply unit according to any one of Technical solutions 9 to 22.
Technical solution 24: The micro-droplet ejection generating device according to technical solution 23,
   The fluid supply unit is detachably connected to the housing.
Technical solution 25: The micro-droplet ejection generating device according to technical solution 23 or 24, further comprising a sensor and a controller, wherein:
   The sensor is used to detect whether the shielding mechanism is opened, and transmits a detection signal to the controller. When the shielding mechanism is opened, the controller controls the piezoelectric actuator to be energized to generate vibration, and then drives the energy transmission sheet to vibrate.
Technical solution 26: The micro-droplet ejection generating device according to any one of technical solutions 23 to 25,
   The micro-nozzle sheet, the energy conducting sheet and the piezoelectric actuator are arranged coaxially, and the distance between the micro-nozzle sheet and the energy conducting sheet is 0 to 3 mm.
Technical solution 27: The micro-droplet ejection generating device according to technical solution 26,

The distance between the micro-nozzle sheet and the energy transmission sheet is 0.2 ∼ 0.8mm

Finally, it should be noted that in the present invention, relational terms such as left and right, first and second, etc. are only used to distinguish one entity or operation from another entity or operation, and not necessarily Requires or implies any such actual relationship or order between these entities or operations. Moreover, the terms "including", "comprising", or any other variation thereof are intended to encompass non-exclusive inclusion, such that a process, method, article, or device that includes a series of elements includes not only those elements but also those that are not explicitly listed or other elements inherent to such a process, method, article, or device. Without more restrictions, the elements defined by the sentence "including a ..." do not exclude the existence of other identical elements in the process, method, article, or equipment including the elements.

Although the present invention has been disclosed above through the description of the specific embodiments of the present invention, it should be understood that those skilled in the art can design various modifications, improvements or equivalents to the present invention within the spirit and scope of the appended claims. These modifications, improvements or equivalents should also be considered to be included in the scope of protection of the present invention.

## Claims

1. A micro-droplet ejection driving device, comprising:
a housing, in which a fluid supply unit may be accommodated;
a piezoelectric actuator, which is fixed on said housing and which is configured to be in close contact with an outer wall of said fluid supply unit and drive said outer wall to vibrate.

2. The micro-droplet ejection driving device according to claim 1, wherein said piezoelectric actuator is a standing wave piezoelectric actuator.

3. The micro-droplet ejection driving device according to claim 1, wherein:
said piezoelectric actuator is a cymbal-liked piezoelectric actuator.

4. The micro-droplet ejection driving device according to claim 2, wherein:
the standing wave piezoelectric actuator comprises a piezoelectric driving element and an energy horn, and the energy horn comprises a coaxial disk segment and a cylindrical segment, the first end surface of the disk segment is connected to the piezoelectric driving element, the other end face of the cylindrical segment is used for close contact with the fluid supply unit.

5. The micro-droplet ejection driving device according to claim 4, wherein:
the cylindrical section and the disc section transition with a concave rounded portion.

6. The micro-droplet ejection driving device according to any one of claims 1 to 5, further comprising:
a guide light source is used for guiding and positioning an ejection target.

7. The micro-droplet ejection driving device according to any one of claims 1 to 5, further comprising:
a shielding mechanism provided on the housing is used to open or close a fluid ejection portion of the fluid supply unit.

8. The micro-droplet ejection driving device according to claim 7, wherein:
the shielding mechanism is an outer cover comprising an elastic structure, which utilizes the elasticity of the elastic structure to open or close the fluid ejection portion.

9. A fluid supply unit comprising:
a fluid ejection portion and an energy conducting sheet,
wherein, the fluid ejection portion and the energy conducting sheet constitute at least part of a container wall of a container for a fluid to be ejected; and
the energy conducting sheet is used to closely contact with an end surface of a piezoelectric actuator and be driven to generate vibration, so that the fluid is ejected through the fluid ejection portion to form a directional micro-droplet stream.

10. The fluid supply unit according to claim 9, further comprising:
an interface, used to communicate with a fluid storage unit.

11. The fluid supply unit according to claim 10, wherein:
the interface comprises a connection pressing member for guiding the fluid storage unit to form a closed connection with the container for the fluid to be ejected.

12. The fluid supply unit according to claim 11, wherein:
the interface also comprises a puncture portion for puncturing the fluid storage unit to achieve communication.

13. The fluid supply unit according to any one of claims 9 to 12, wherein:
the fluid ejecting portion and the energy conducting sheet are oppositely arranged on the container for the fluid to be ejected.

14. The fluid supply unit according to any one of claims 9 to 12, wherein:
the fluid ejection portion is a micro-nozzle sheet, and the micro-nozzle sheet has at least one nozzle hole.

15. The fluid supply unit according to claim 14, wherein:
the nozzle holes are distributed in a region of 1 mm to 10 mm in diameter with the center as the origin on the micro-nozzle sheet, and the diameter of the nozzle holes is 5 µm to 200 µm.

16. The fluid supply unit according to claim 14, wherein:
the diameter of a fluid inlet end of the nozzle holes is larger than the diameter of a fluid outlet end of the nozzle holes.

17. The fluid supply unit according to claim 16, wherein:
the nozzle hole is a tapered hole, and its side wall is curved toward the center of the nozzle hole to form an arc-shaped wall.

18. The fluid supply unit according to claim 14, wherein:
the sidewall surface of the nozzle hole has a hydrophobic layer or a hydrophilic layer.

19. The fluid supply unit according to claim 18, wherein:
the hydrophobic layer or the hydrophilic layer is obtained by material modification of the sidewall surface of the nozzle hole.

20. The fluid supply unit according to any one of claims 9 to 12, wherein further comprising: a U-shaped groove, which is provided on an inner wall surface of the container for the fluid to be ejected, and is located on the periphery of the fluid ejection portion, and is used to provide a storage space for small volume of fluid.

21. The fluid supply unit according to any one of claims 9 to 12, wherein:
the energy conducting sheet is made of a metal sheet with a thickness of 0.01mm to 0.2mm.

22. The fluid supply unit according to any one of claims 9 to 12, wherein:
the energy conducting sheet is made of non-metallic film with a thickness of 0.01mm to 0.1mm.

23. A micro-droplet ejection generating device, comprising said micro-droplet ejection driving device according to any one of claims 1 to 8 and said fluid supply unit according to any one of claims 9 to 22.

24. The micro-droplet ejection generating device according to claim 23, wherein:
said fluid supply unit is detachably connected to the housing.

25. The micro-droplet ejection generating device according to claim 23 or 24, further comprising a sensor and a controller, wherein,
the sensor is used to detect whether the shielding mechanism is opened, and transmit the detection signal to the controller; when the shielding mechanism is opened, the controller controls said piezoelectric actuator to vibrate, thereby driving the energy conducting sheet to vibrate.

26. The micro-droplet ejection generating device according to claim 23 or 24, wherein:
said micro-nozzle sheet, said energy conducting sheet and said piezoelectric actuator are arranged coaxially, and the distance between said micro-nozzle sheet and said energy conducting sheet is 0 to 3 mm.

27. The micro-droplet ejection generating device according to claim 26, wherein:
the distance between said micro-nozzle sheet and said energy conducting sheet is 0.2 ∼ 0.8mm.
